# EUROPEAN PATENT SPECIFICATION

(11) **EP 3 046 498 B1**
(45) Date of publication and mention of the grant of the patent: **23.08.2017**
(21) Application number: 14781278.8
(22) Date of filing: 01.09.2014
(51) Int. Cl.: A61B 90/70, A47L 15/22, B08B 3/02

(54) **ROTATING WASH GROUP**
ROTIERENDE WASCHBAUGRUPPE
GROUPE DE LAVAGE ROTATIF

(30) Priority: 18.09.2013 SI 201300278
(43) Date of publication of application: 27.07.2016
(73) Proprietor: Belimed AG, 6301 Zug (CH)
(72) Inventor: PETRIC, Samo, 1386 Stari trg pri Lozu (SI)
(74) Representative: Hepp Wenger Ryffel AG
(86) International application number: PCT/IB2014/064162
(87) International publication number: WO 2015/040515

(56) References cited:
- EP-A1- 1 190 665
- EP-A1- 1 949 868
- DE-A1- 1 403 630
- US-A1- 2004 118 440

## Description

The present invention relates to a washing machine in a sterilisation section of a hospital for washing and/or sterilisation of a surgical instruments, for instance, and similar, and in particular to an improved rotating wash group of said washing machine.

A washing machine of the aforementioned kind is known from a patent application No. EP 1 190 665 A1. Disclosed is a rotary nozzle for washing machines comprising at least a chamber inside which objects to be washed are placed. Said nozzle being able to deliver towards said objects a liquid detergent and comprising a first fixed component to feed and distribute said liquid detergent and a second rotary component to deliver said liquid detergent, said first component being able to be coupled inside said second component and including spraying means able to deliver pressurized fluid towards mating hollows made tangentially inside said second component to impart thereto a thrust which makes it rotate on its own longitudinal axis. However, said known solution is intended for washing hollow containers from the inside to the outside, and is rather impractical for washing small and difficult to clean objects .

The two part form of claim 1 is based on EP 1949868 A1. It is the object of the present invention to create a wash group particularly with a washing machine in a sterilisation section of a hospital for washing and/or sterilisation of surgical instruments, for instance, and similar, which remedies drawbacks of know solutions.

The object as set above is solved with the characteristics disclosed in a characterizing clause of the claim 1. Detail of the invention is revealed in the corresponding subclaims.

The invention is further described in detail by way of non-limiting embodiment, and with a reference to the accompanying drawings, where
- Fig. 1: shows a three-dimensional exploded view of a rotating wash group according to the invention,
- Fig. 2: shows a cross-sectional view of the assembled rotating wash group of Fig. 1 through the longitudinal vertical plane,
- Fig. 3: shows a cross-sectional view of the assembled rotating wash group of Fig. 1 through the transversal vertical plane,
- Fig. 4: shows detail **IV** of Fig. 3,
- Fig. 5: shows another embodiment of detail of Fig. 4.

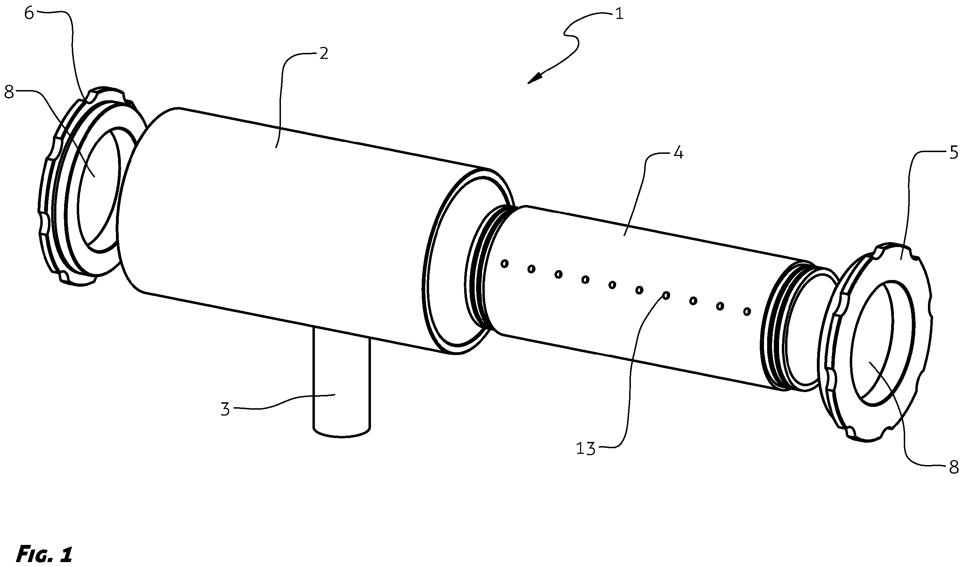

Fig. 1 shows a three-dimensional exploded view of a rotating wash group 1 of a washing machine in particular a washing machine in a sterilisation section of a hospital for washing and/or sterilisation of a surgical instruments, for instance, and similar, comprising a tubular body 2 with an inlet projection 3 for a washing agent, a tubular washing rotor 4 arranged in a rotatable manner in the body 2, and holding flanges 5, 6 to secure the rotor 4 in said body 2. Said body 2 and said rotor 4 are arranged approximately coaxial. Said inlet projection 3 is arranged eccentrically to the body 2, viewed in the transversal direction thereof. Each flange 5, 6 is formed with a centric radially extending cylindrical projection 7 provided with a centric through-hole 8. The external circumference of said projection 7 is connected by means of a temporary joint, such as a threaded joint, with each end of the body 2. Said tubular rotor 4 arranged in the body 2 is formed in the area of its each end with a radially extending step 9 with which cooperates each flange 5, 6 pressing against said step 9, thus, fixing the rotor 4 in the body 2. It is obviously, of course, that the connection between each step 9 of the rotor 4 and each flange 5, 6 is formed with a sliding fit in order for the rotor 4 to rotate in said flanges 5, 6. Thus, a substantial amount of space 10 is provided in the described manner between said body 2 and said rotor 4, said space 10 being filed with a washing agent during operation of the wash group 1 according to the invention.

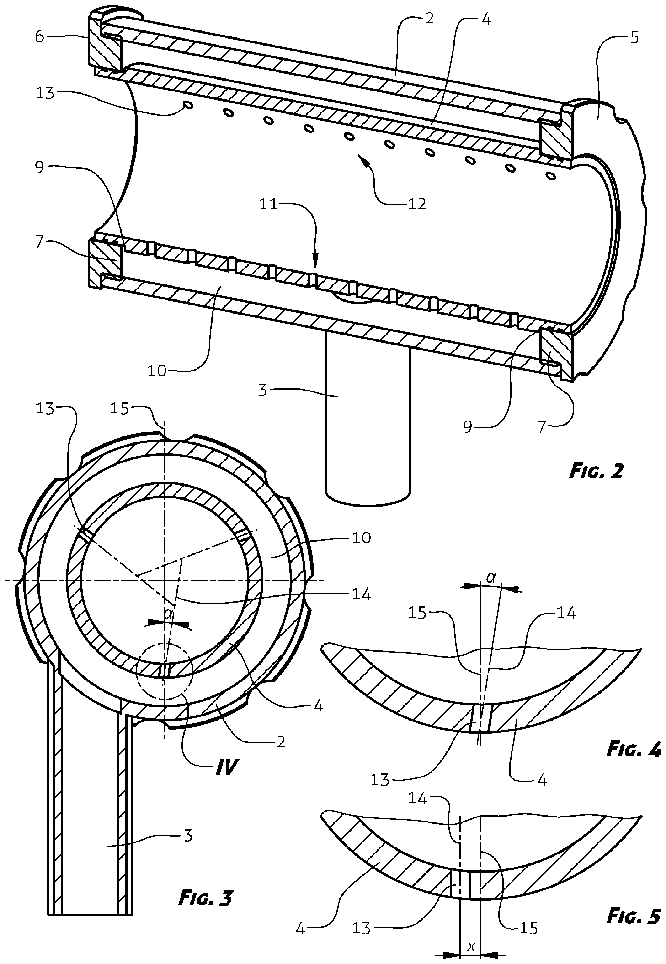

Furthermore, said rotor 4 is formed over its circumference with at least two sets 11, 12 of through-holes 13 to supply the washing agent from said space 10 into the tubular rotor 4. Each said set 11, 12 of through-holes 13 lies approximately in the longitudinal direction of the rotor 4 with regard to the body axis of the rotor 4, however, other extends are possible such as in the sense of a helical curve, a spiral and the like. Each hole 13 is formed in the cross-sectional view of the assembly 1 (Fig. 3, Fig. 4) in a manner that a body axis 14 of the hole 13 is inclined for the angle *α* with regard to the transversal body axis 15 of the rotor 4, said angle *α* ranges between 15° in 45°. Further possibility provides a form of each hole 13 where its body axis 14 extends in parallel to the body axis 15 of the rotor 4 yet the body axis 14 of the hole 13 is formed so as to be spaced for the value x from the body axis 15 of the rotor 4 (Fig. 5). It is, however, obvious that also an arbitrary combination of the above described arrangement of the holes 13 is possible without departing from the scope of the invention.

During operation of a washing machine in particular a washing machine in a sterilisation section of a hospital for washing and/or sterilisation of a surgical instruments, for instance, and similar, the surgical instrument to be washed is inserted into the rotating wash group 1 according to the invention, in particular into the central section of the tubular rotor 4. Afterwards, the pressurized washing agent is dispensed via said inlet projection 3 into the space 10 between the body 2 and the rotor 4, said washing agent passes through the set 11, 12 of the through-holes 13 from the space 10 to the interior of the tubular rotor 4. Due to said inclination *α* of the holes 13 and, respectively, due to said spacing x of the holes 13, and due to said eccentric arrangement of the inlet projection 3 the washing agent that streams in exerts a tangential fluid load to the walls of the holes 13 resulting in a rotational movement of the rotor 4. The washing agent that streams via holes 13 in the interior of the rotor 4 sprays through said holes 13 and the resulting jets intersect in the central section of the rotor 4 and hit the surgical instrument to be washed over the entire length of the rotor 4 and, respectively, the length of the set 11, 12 of the holes 13. Thus, an excellent cleaning effect is achieved since the jets of the washing agent reach all the places on the surgical instrument to be washed.

## Claims

1. A wash group of a washing machine such as a washing machine in a sterilisation section of a hospital for washing and/or sterilisation of a surgical instrument for instance, and similar, comprising a tubular body (2) with an inlet projection (3) for a washing agent, **characterised in that** said wash group further comprises a tubular washing rotor (4) having a set (11, 12) of through-holes (13) arranged in a rotatable manner inside the tubular body (2), and holding flanges (5, 6) to secure the rotor (4) in said body (2).

2. A rotating wash group according to claim 1, ***characterised in that*** said rotor (4) is formed over its circumference with at least two sets (11, 12) of through-holes (13) to supply the washing agent from a space (10) between the body (2) and the rotor (4) into the tubular rotor (4), each said set (11, 12) of the holes (13) extends approximately in longitudinal direction of the rotor (4) with regard to the body axis of the rotor (4).

3. A rotating wash group according to claims 1 and 2, ***characterised in that*** each said set (11, 12) of holes (13) extends in the sense of a helical curve, a spiral and the like with regard to the body axis of the rotor (4).

4. A rotating wash group according to claims 1 to 3, ***characterised in that*** each hole (13) is formed in a manner that its body axis (14), viewed in a cross-section of the group (1), is formed in a manner that its body axis (14) is inclined for an angle *α* with regard to a transversal body axis (15) of the rotor (4).

5. A rotating wash group according to claim 4, ***characterised in that*** the value of the angle *α* ranges between approximately 15° and approximately 45°.

6. A rotating wash group according to claims 1 to 3, ***characterised in that*** each hole (13) is formed in a manner that its body axis (14) extends in parallel with the body axis (15) of the rotor (4), said body axis (14) of the hole (13) being formed so as to be spaced for the value x from the body axis (15) of the rotor (4).

7. A rotating wash group according to any of the preceding claims, ***characterised in that*** said body (2) and said rotor (4) are arranged approximately coaxial.

8. A rotating wash group according to any of the preceding claims, ***characterised in that*** said inlet projection (3) is arranged eccentrically with regard to the body (2) when viewed transversally.

## Patentansprüche

1. Waschbaugruppe einer Waschmaschine wie z. B. einer Waschmaschine in einem Sterilisationsabschnitt eines Krankenhauses zum Waschen und/oder Sterilisieren von beispielsweise einem chirurgischen Instrument und dergleichen, umfassend
einen rohrförmigen Körper (2) mit einem Einlassvorsprung (3) für ein Waschmittel, **dadurch gekennzeichnet, dass** die Waschbaugruppe ferner einen rohrförmigen Waschrotor (4), der einen Satz (11, 12) von Durchgangslöchern (13) aufweist und drehbar im rohrförmigen Körper (2) angeordnet ist, und Halteflansche (5, 6) zum Sichern des Rotors (4) im Körper (2) umfasst.

2. Rotierende Waschbaugruppe nach Anspruch 1, **dadurch gekennzeichnet, dass** der Rotor (4) über ihren Umfang mit mindestens zwei Sätzen (11, 12) von Durchgangslöchern (13) zum Zuführen des Waschmittels aus einem Raum (10) zwischen dem Körper (2) und dem Rotor (4) in den rohrförmigen Rotor (4) ausgebildet ist, wobei sich jeder Satz (11, 12) von Löchern (13) in etwa in Längsrichtung des Rotors (4) im Hinblick auf die Körperachse des Rotors (4) erstreckt.

3. Rotierende Waschbaugruppe nach einem der Ansprüche 1 bis 2, **dadurch gekennzeichnet, dass** sich jeder Satz (11, 12) Löcher (13) entlang einer schraubenförmigen Kurve, einer Spirale und dergleichen in Bezug auf die Körperachse des Rotors (4) erstreckt.

4. Rotierende Waschbaugruppe nach einem der Ansprüche 1 bis 3, **dadurch gekennzeichnet, dass** jedes Loch (13) derart ausgebildet ist, dass seine Körperachse (14) im Querschnitt durch die Baugruppe (1) derart gebildet ist, dass die Körperachse (14) mit einem Winkel α in Bezug auf eine quer verlaufende Körperachse (15) des Rotors (4) geneigt ist.

5. Rotierende Waschbaugruppe nach Anspruch 4, **dadurch gekennzeichnet, dass** der Wert des Winkels α zwischen etwa 15° und etwa 45° liegt.

6. Rotierende Waschbaugruppe nach einem der Ansprüche 1 bis 3, **dadurch gekennzeichnet, dass** jedes Loch (13) derart ausgebildet ist, dass sich seine Körperachse (14) parallel zur Körperachse (15) des Rotors (4) erstreckt, wobei die Körperachse (14) des Lochs (13) derart ausgebildet ist, dass sie mit einem Wert x von der Körperachse (15) des Rotors (4) beabstandet ist.

7. Rotierende Waschbaugruppe nach einem der vorhergehenden Ansprüche, **dadurch gekennzeichnet, dass** der Körper (2) und der Rotor (4) in etwa koaxial angeordnet sind.

8. Rotierende Waschbaugruppe nach einem der vorhergehenden Ansprüche, **dadurch gekennzeichnet, dass** der Einlassvorsprung (3) im Hinblick auf den Körper (2) aus Queransicht exzentrisch angeordnet ist.

## Revendications

1. Groupe de lavage d'une machine à laver telle qu'une machine à laver dans une section de stérilisation d'un hôpital pour le lavage et/ou la stérilisation d'un instrument chirurgical, par exemple, et similaires, comprenant un corps tubulaire (2) avec une saillie d'entrée (3) pour un agent de lavage, **caractérisé en ce que** ledit groupe de lavage comprend en outre un rotor de lavage tubulaire (4) possédant un ensemble (11, 12) de trous débouchants (13) agencé de manière rotative à l'intérieur du corps tubulaire (2), et des brides de retenue (5, 6) pour fixer le rotor (4) dans ledit corps (2).

2. Groupe de lavage rotatif selon la revendication 1, **caractérisé en ce que** ledit rotor (4) est formé sur sa circonférence avec au moins deux ensembles (11, 12) de trous débouchants (13) pour fournir l'agent de lavage à partir d'un espace (10) entre le corps (2) et le rotor (4) dans le rotor tubulaire (4), chaque dit ensemble (11, 12) des trous (13) s'étend approximativement dans la direction longitudinale du rotor (4) par rapport à l'axe de corps du rotor (4).

3. Groupe de lavage rotatif selon les revendications 1 et 2, **caractérisé en ce que** chaque dit ensemble (11, 12) de trous (13) s'étend dans le sens d'une courbe hélicoïdale, d'une spirale, et analogue, par rapport à l'axe de corps du rotor (4).

4. Groupe de lavage rotatif selon les revendications 1 à 3, **caractérisé en ce que** chaque trou (13) est formé de manière telle que son axe de corps (14), en vue en section transversale du groupe (1), soit formé d'une manière telle que son axe de corps (14) soit incliné selon un angle *α* par rapport à un axe de corps transversal (15) du rotor (4).

5. Groupe de lavage rotatif selon la revendication 4, **caractérisé en ce que** la valeur de l'angle *α* varie entre approximativement 15° et approximativement 45°.

6. Groupe de lavage rotatif selon les revendications 1 à 3, **caractérisé en ce que** chaque trou (13) est formé de manière telle que son axe de corps (14) s'étende en parallèle avec l'axe de corps (15) du rotor (4), ledit axe de corps (14) du trou (13) étant formé afin d'être espacé, selon la valeur x, de l'axe de corps (15) du rotor (4).

7. Groupe de lavage rotatif selon l'une quelconque des revendications précédentes, **caractérisé en ce que** ledit corps (2) et ledit rotor (4) sont agencés de façon approximativement coaxiale.

8. Groupe de lavage rotatif selon l'une quelconque des revendications précédentes, **caractérisé en ce que** ladite saillie d'entrée (3) est agencée de façon excentrique par rapport au corps (2) en vue transversale.
